# EUROPÄISCHE PATENTANMELDUNG

(11) **EP 1 053 758 A1**
(43) Veröffentlichungstag der Anmeldung: **22.11.2000**
(21) Anmeldenummer: 99109831.0
(22) Anmeldetag: 19.05.1999
(51) Int. Cl.: A61L 27/48, A61L 31/12

(54) **Bioabsorbierbares Implantat**

(71) Anmelder: Resorba Chirurgisches Nahtmaterial Franz Hiltner GmbH & Co., 90443 Nürnberg (DE)
(72) Erfinder: Hiltner, Claus Martin, 90408 Nürnberg (DE)
(74) Vertreter: Schneck, Herbert, Dipl.-Phys., Dr.

(57) **Zusammenfassung**

Bei einem bioabsorbierbaren Implantat ist vorgesehen, daß in eine Grund-matrix aus biologisch kompatiblem Material, wie Kollagen, Gelatine, Methylzellulosederivaten, Hyaluronsäurederivaten, Alginaten oder Mischungen aus verschiedenen genannten Stoffen und dergleichen ein Faser-Stützgerüst aus bioabbaubaren Polymeren, wie Polyglykolsäure, Polyglykolid sowie deren Copolymeren und Polycaprolacton oder nicht resorbierbarer Fasern wie Polypropylen, Polyester oder Polyvinylidenfluorid eingebaut ist.

## Beschreibung

Die Erfindung richtet sich auf ein bioabsorbierbares Implantat.

Derartige Implantate sind seit vielen Jahren in unterschiedlicher Form, aus unterschiedlichem Material und verschiedene Zweckbestimmungen im klinischen Einsatz. Eine typische Form stellt z.B. die Anwendung von Wundund Gewebeeinlagen auf der Grundlage von Kollagen, Methylzellulose bzw. oxidierter Methyl-Zellulose oder bioabbaubaren Polymeren dar. Gemeinsames Ziel ist stets die Übernahme einer temporären Festigungs- oder Platzhaltefunktion bis nach Abschluß des Heilungsvorganges eigenes Körpergewebe die Funktion wieder gewährleistet. Daneben kommen auch Aspekte der lokalen Blutstillung bei chirurgischen Eingriffen oder die lokale Wirkstoffapplikation hinzu. Ein Beispiel hierfür sind Implantate aus Kollagen als Lyphilisat oder Film zur lokalen hämostatischen Wundbehandlung, die durch Freisetzung von antibiotisch wirksamen Stoffen daneben bakteriostatische Wirkung entfalten.

Die verschiedenen in Betracht kommenden Stoffgruppen unterscheiden sich teilweise erheblich einerseits in ihren mechanischen Eigenschaften und andererseits in ihrer Biokompatibilität.

So werden beispielsweise chirurgische Netzimplantate aus bioabbaubaren Polymeren nicht bei jeder prinzipiell möglichen Indikation eingesetzt, obwohl sie aufgrund ihrer mechanischen Eigenschaften geeignet erscheinen, da der Abbau der Polymerfasern z.B. aus Polyglykol-Polylactid oder Polycaprolacton im Gewebe zu einer lokalen Verschiebung des pH-Wertes führt, wodurch unerwünschte Abwehrreaktionen hervorgerufen werden können. Dieses Phänomen ist unsystematisch individuell unterschiedlich stark geprägt und scheint von der Größe des Implantats sowie dem Einsatzort im Körper abzuhängen und führt nicht selten zu einem Verlust des Implantates, wodurch der therapeutische Erfolg in Frage gestellt wird bzw. zusätzliche chirurgische Interventionen bzw. Nachsorgebehandlungen nötig werden. Grundsätzlich sind im Sinne der Risikoabwägung Biokompatibilitätsprobleme von Implantaten (Medizinprodukten) stets weitestgehend auszuschließen, jedoch bei keinem körperfremdem, insbesondere synthetischen Material vollständig zu vermeiden. Es gilt daher für jede Indikation eine entsprechende Risikoeinschätzung bezüglich Implantationsart, Implantatart und Größe sowie Implantationsdauer vorzunehmen. Dementsprechend werden Implantate mit erhöhten Biokompatibilationsrisiken, wenn möglich nicht dauerhaft im Körper belassen, sondern wieder explantiert, was naturgemäß mit einer erheblichen Belastung des Patienten verbunden ist.

Eine traditionelle Indikation für chirurgische Netze stellt die sehr weit verbreitete Hernienchirurgie dar, bei der aus den genannten Gründen heute vor allem nichtresorbierbare Netze aus Polypropylen bzw. Polyester eingesetzt werden. Diese Implantate weisen zwar hervorragende mechanische Eigenschaften auf, sind jedoch obwohl grundsätzlich nicht resorbierbar nicht vollständig chemisch inert und können sowohl eine Abwehrreaktion im Gewebe auslösen als auch langfristig degradativ verspröden. Diese Reaktionen gehen häufig mit einer bindegewebsartigen Einkapselung des Implantates und Gewebeverhärtungen einher.

Demgegenüber weisen beispielsweise bioabbaubare Kollagene, ebenso wie Gewebe aus Methylzellulose eine hervorragende Biokompatibilität auf, wobei deren mechanische Festigkeit aber geringer ist, so daß sie in Indikationsbereichen mit erhöhter mechanischer Beanspruchung nicht eingesetzt werden können. Insbesondere sind diese Implantate aufgrund ihrer Resorptionseigenschaften für dauerhafte Stütz- und Haltefunktionen ungeeignet.

Hiervon ausgehend liegt der Erfindung die Aufgabe zugrunde, ein Implantat der eingangs genannten Art so auszugestalten, daß es bei hoher biologischer Kompatibilität auch höheren mechanischen Anforderungen gerecht wird.

Diese Aufgabe wird erfindungsgemäß dadurch gelöst, daß in eine Grundmatrix aus biologisch kompatiblem Material, wie Kollagen, Gelatine, Methylzellulosederivaten, Hyaluronsäurederivaten oder Alginaten und dergleichen ein Faser-Stützgerüst entweder aus bioabbaubaren Polymeren, wie Polyglykolsäure, Polyglykolid sowie deren Copolymeren oder Polycaprolactonfasern bzw. ein entsprechendes Gerüst aus nicht resorbierbaren Fasern aus Polypropylen, Polyester oder Polyvinylidenfluorid eingebaut ist.

Das Stützgerüst kann in Form von Fasern, Fasergewirken oder Netzen ausgebildet sein. Die Implantate als solche können als Schwämme (in Form von Wundeinlagen), Filme (zur Realisierung einer Abdeckfunktion z.B. als temporärer Hirnhautersatz), Stränge (in Form von Sehnen bzw. Faszienersatz) oder Blöcken (zur Knochenintegration) ausgebildet sein.

In jedem Fall sind die Fasergerüste von einer Schicht biologisch hochkompatibler Stoffe umgeben, die die Integration in das einzuwachsende Wundgewebe fördert. Bekannt ist z.B. die Leitschienenfunktion von Kollagen für das Einwachsen von Gewebezellen.

Je nach Indikationsstellung kann das resorbierbare Implantat mit einer Armierungsstruktur zur dauerhaften Stützfunktion (z.B. Polypropylen) aus einer nicht resorbierbaren Faser oder zur temporären Stützfunktion mit einem resorbierbaren Fasermaterial ausgestattet werden.

Die so gebildeten Verbundimplantate können in einer weiteren Funktion auch mit pharmakologisch wirksamen Stoffen, wie z.B. Antibiotika, zur Gewährleistung einer lokalen antibiotischen Prophylaxe zum Schutz vor Infektion des Wundgebietes ausgestattet werden,.

Der Wirkstoff wird dabei unmittelbar während und nach der Implantation durch den Kontakt des Implantates mit Körperflüssigkeiten freigesetzt und entfaltet eine lokale prophylaktische Antibiose.

Das Verhindern einer Infektion stellt damit einen zusätzlichen Schutz für den Verlust des Implantates und damit aufwendiger weiterer Wundversorgung/Sanierung dar.

## Patentansprüche

1. Bioabsorbierbares Implantat, **dadurch gekennzeichnet, daß** in eine Grundmatrix aus biologisch kompatiblem Material, wie Kollagen, Gelatine, Methylzellulosederivaten, Hyaluronsäurederivaten, Alginaten oder Mischungen aus verschiedenen genannten Stoffen und dergleichen ein Faser-Stützgerüst aus bioabbaubaren Polymeren, wie Polyglykolsäure, Polyglykolid sowie deren Copolymeren und Polycaprolacton oder nicht resorbierbarer Fasern wie Polypropylen, Polyester oder Polyvinylidenfluorid eingebaut ist.

2. Implantat nach Anspruch 1, **dadurch gekennzeichnet, daß** das Stützgerüst in Form von Fasern, Fasergewirken oder Netzen ausgebildet ist.

3. Implantat nach Anspruch 1 oder 2, **dadurch gekennzeichnet, daß** das Implantat als Schwamm, Film, Strang, Platte, Stift oder Block ausgebildet ist.

4. Implantat nach den Ansprüchen 1 bis 3, **dadurch gekennzeichnet, daß** ein pharmabiologisch wirksamer Stoff zur lokalen antibiotischen Prophylaxe oder Behandlung in die Grundmatrix eingebracht ist.

5. Implantat nach den Ansprüchen 1 bis 3, **dadurch gekennzeichnet, daß** es einen oder mehrere wachstumsfördernde oder zellintegrationsfördernde Faktoren, wie rekombinante Wachtumsfaktoren für Stützgewebe oder zur Förderung der Revascularisierung enthält.
